(19) Europäisches Patentamt / European Patent Office / Office européen des brevets

(11) **EP 2 954 935 A1**

(12) **EUROPEAN PATENT APPLICATION**

(43) Date of publication:
**16.12.2015 Bulletin 2015/51**

(51) Int Cl.:
**A61Q 13/00** (2006.01)    **A61K 8/03** (2006.01)
**A61K 8/31** (2006.01)    **A61K 8/34** (2006.01)

(21) Application number: **14172212.4**

(22) Date of filing: **12.06.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Coty Inc.**
**New York, NY 10118 (US)**

(72) Inventors:
• **Bleuez, Loic**
 **01210 Ornex (FR)**

• **Du Theil, Clelia Martin**
 **1206 Genève (CH)**
• **Abriat, Anne**
 **1202 Genève (CH)**
• **Vernaz, Philippe Dominique**
 **01280 Prévessin-Moens (FR)**
• **Smith, Leslie, Dr.**
 **1263 Crassier (CH)**

(74) Representative: **Gulde & Partner**
**Patent- und Rechtsanwaltskanzlei mbB**
**Wallstraße 58/59**
**10179 Berlin (DE)**

(54) **Biphasic composition for perfume and personal care applications and uses thereof**

(57)    The present invention relates to a biphasic composition comprising or consisting of alcohol, a polar compound, a fragrance and a branched hydrocarbon.

EP 2 954 935 A1

**Description**

The present invention relates to a biphasic composition. Specifically, the present invention relates to a biphasic composition comprising or consisting of alcohol, water, a fragrance and a branched hydrocarbon.

Fragrancing or perfuming of products has been known for a long time. Fragrances or perfumes are frequently incorporated in cosmetic compositions to give them a pleasant odor that is also transferred to the skin, hair or clothes of the user. Fragrances or perfumes in cosmetic, toiletry and personal care applications provide several functions. They mask base odors, provide an olfactory aesthetic benefit and serve as a signal of product attributes and function, e.g. hygiene and cleanliness.

Products comprising fragrances or perfumes can be categorized into different types based on the concentration of fragrances within those products. The intensity and longevity of such products is based on the concentration, intensity and longevity of the fragrances which are used; the higher the percentage of fragrances, the more intense and long-lasting the scent of the product is. Specific terms are used to describe a perfumed product's approximate concentration by volume% of fragrance. Examples are perfume extract with 15-40% fragrance, Esprit de Parfum (ESdP) with 15-30% fragrance, Eau de Parfum (EdP) with 10-20% fragrance, Eau de Toilette (EdT) with 5-15% fragrance and splash, Eau de soin or aftershave with 1-4% fragrance.

Upon storage, fragrances and perfumes can be altered through interactions and/or reactions with the other components of the composition which may lead to a loss of fragrance intensity and/or an unwanted change of the scent.

The fragrances or cosmetic compositions described until now usually constitute of mixtures of different fragrancing substances forming an integral fragrance or perfume. However, to avoid unwanted reactions between the different components wherever possible, alternative compositions are needed that exhibit a suitable constitution. Furthermore, it is advantageous if the composition additionally has a unique and appealing appearance to attract the consumer's attention.

It is an object of the present invention to provide alternatives to known cosmetic compositions.

The present invention provides a biphasic composition comprising or consisting of alcohol, water, a fragrance and a branched hydrocarbon.

The biphasic composition of the present invention is a cosmetic composition.

It has been found that it is possible to create a biphasic composition that has different olfactory and/or colored characteristics in the individual phases of the composition.

As used in this specification and the appended claims, the singular forms "a", "an" and "the" include singular and plural referents unless the content clearly dictates otherwise. Thus, for example, reference to "a hydrocarbon" includes one hydrocarbon and a combination of two or more hydrocarbons, and the like.

In the present invention, the term "biphasic" is intended to mean at least two distinct and homogeneous forms of matter, preferably of liquids, that are separated from each other by their surfaces when left to stand. Biphasic compositions are constituted in general by a distinct aqueous and a distinct oily phase. Usually, the aqueous phase forms the bottom or lower phase and the oily phase forms the upper or top phase. According to the present invention, the distinct phases are transparent when left to stand. When the composition is agitated prior to use, the mixture obtained has an opaque character.

The two phases may be layered with one phase on top of the other. However, the two phases may also be arranged in an alternative way e.g. that one phase may form spherical or oval shapes within the other phase.

Alternatively, it is possible to amend the composition in a way that a modification of the density of one phase, e.g. the oily phase, leads to a change of its physical properties within the biphasic composition. The density is a physical quantity defined by the following formula:

$$\rho = \frac{m}{V}$$

wherein p is the density, m is the mass and V is the volume. The SI-unit for density is $kg/m^3$. The person skilled in the art knows what the density means or how it can be determined. Depending on the selected density of the one phase, the two phases of the biphasic composition need not to be layered but instead it is possible that the one phase may form shapes within the other phase, e.g. the oily phase may form a spherical or oval shape within the aqueous phase. Preferably, the oily phase has a density from 0.8033 to 0.866 $g/cm^3$. According to the present invention, the aqueous phase is transparent and the oily phase is cloudy when left to stand. When the composition is agitated prior to use, the mixture obtained has a transparent character with cloudy spherical or oval forms in it or, when the composition is agitated more vigorously, the mixture has a slightly cloudy character.

The biphasic composition of the present invention contains a polar compound. As used herein, the term "polar compound" denotes a substance consisting of a polar molecule having an electric dipole moment that is formed by the partial positive and partial negative charge of the polar molecule. Due to the polar nature, polar compounds are generally able to dissolve in polar solvents. Whether a compound disperses rather in a polar, e.g. aqueous, solvent or in an unpolar, e.g. oily, solvent depends on its solubility. Solubility is the property of a compound (i.e. the solute) to dissolve in a solvent to form a homogeneous solution of the solute in the solvent. The solubility of a compound depends on the physical and chemical properties of the compound and solvent as well as on temperature, pressure and the pH of the solution. In order to calculate the solubility of a compound, it is possible to determine the molecule's electro negativity difference ($\Delta$EN) which is the difference between the electro negativities of the atoms that are involved in a chemical bond. Generally, if $\Delta$EN of a molecule or compound is between 0.5 and 1.7 the molecule or compound is polar and therefore water-soluble. If $\Delta$EN of a molecule or compound is < 0.5 the molecule or compound is non-polar and therefore lipid-soluble. The person skilled in the art knows what the $\Delta$EN mean or how it can be determined.

Another important factor is the partition coefficient of a compound which is the ratio of concentrations of the compound in the two phases of a mixture of two immiscible liquids (e.g. solvents) at equilibrium. The partition coefficient is a measure of how hydrophilic/lipophobic or hydrophobic/lipophilic the compound is. Normally one of the solvents is water while the second is hydrophobic such as 1-octanol. The logarithm of the ratio of the concentrations of the compound in the solvents is called log P which is also known as a measure of lipophilicity and is represented by the following formula:

$$\log P_{\text{oct/wat}} = \log \left( \frac{[\text{solute}]_{\text{octanol}}}{[\text{solute}]_{\text{water}}^{\text{un-ionized}}} \right)$$

Compounds with high octanol/water partition coefficients (> 1.00) are preferentially found in the hydrophobic phase which corresponds to the oily phase. Compounds with low octanol/water partition coefficients (< 1.00) preferentially are found in the hydrophilic phase which corresponds to the water/alcohol phase. This shall be exemplified with the following values: the log P-value of methanol is -0.82 which means it is hydrophilic; the log P-value of isododecan is 6.4 which means it is lipophilic. The person skilled in the art knows what the log P-value mean or how it can be determined.

According to the present invention, the polar compounds have a logP-value < -0.7. Compounds with a logP-value of $\geq$ - 0.7 are most often not suited since they are not polar enough to allow the formation of a biphase composition. In particular, the polar compounds can have a logP-value of -0.7 to -4. Preferred polar compounds are water (logP = -1.38), Sorbitol 70 (logP = -3.01), glycerin (logP = -1.76), PEG-4 (logP = -2.02), PEG-8 (logP = -3.12), propylene glycol (logP = -0.92) and polyols. Preferably, the polar compound is water. More preferably, the ions or ionic constituents have been removed from the water (deionized water) prior using it in the present application. According to the present invention, the biphasic composition may comprise one polar compound or a mixture of two or more polar compounds.

The way how a certain compound dispenses in the aqueous or in the oily phase depends on the percentage of polar compound, e.g. water, within the aqueous/alcoholic phase of the biphasic composition of the invention. Surprisingly, it has been found that by regulating the amount of polar compound in the water/alcohol-phase it is possible to achieve that a defined amount of a compound dispenses in a particular phase.

According to the present invention, the polar compound is contained in the biphasic composition in an amount of 0.5 to 30 wt%, preferably in an amount of 0.75 to 15 wt%, more preferred in an amount of 1 to 10 wt% and even more preferred in an amount of 2 to 9 wt% based on the total weight of the composition.

By adjusting the amount of the polar compound it is possible to regulate the quantity of e.g. the fragrance in a particular phase of the composition of the present invention. This shall be illustrated with the following examples (all data given in wt% refer to the total weight of the respective compound within the composition): if the water/alcohol-phase contains ~ 7.4 wt% water, ~30 wt% of the fragrance is in the upper oily phase and ~70 wt% of the fragrance is in the bottom water/alcohol-phase. If the water/alcohol-phase contains ~ 14 wt% water, ~49 wt% of the fragrance is in the upper oily phase and -51 wt% of the fragrance is in the bottom water/alcohol-phase. If the water/alcohol-phase contains ~ 27.2 wt% water, ~70 wt% of the fragrance is in the upper oily phase and ~30 wt% of the fragrance is in the bottom water/alcohol-phase.

In a preferred embodiment, the biphasic composition comprises water as polar compound. Due to the water content the biphasic composition of the invention shows instant moisturizing properties, hydrates the skin and gives the product freshness.

Preferably, the aqueous phase of the composition is in the form of an aqueous or aqueous-alcoholic solution.

The biphasic composition of the present invention contains alcohol. As used herein the term "alcohol" denotes any organic compound whose molecule contains one or more hydroxyl groups attached to a carbon atom. The alcohol may comprise up to 10% water. Preferably, the alcohol is a mono-valent C2-C5 alcohol. In a preferred embodiment of the invention, the alcohol is ethanol.

According to the present invention, the alcohol is contained in the biphasic composition in an amount of 20 to 75 wt%, preferably in an amount of 30 to 70 wt% and more preferred in an amount of 40 to 65 wt% based on the total weight of the composition.

The biphasic composition of the present invention contains a fragrance. According to the present invention, the fragrance comprises at least a first fragrance and a second fragrance.

The term "fragrance" or "first fragrance" as used in the present invention means an aroma or an aroma compound, odorant or scent which is a natural, semi-synthetic or synthetic compound that has a smell or odor. The fragrance can comprise a mixture of two or more fragrancing substances.

Fragrances can include natural products such as extracts and/or essences, essential oils, balsams, absolutes, resinoids, resins, tinctures, concretes etc., which may comprise complex mixtures of constituents, i.e. fruits such as almond, apple, cherry, grape, pear, pineapple, orange, lemon, strawberry, raspberry and the like; musk, flower scents such as lavender, jasmine, lily, magnolia, rose, iris, carnation and the like; herbal scents such as rosemary, thyme, sage and the like; woodland scents such as pine, spruce, cedar and the like. They may also include synthetic materials such as hydrocarbons, alcohols, aldehydes, ketones, ethers, acids, esters, acetals, ketals, nitriles, etc., including saturated, unsaturated, aliphatic and cyclic compounds.

Examples of fragrance materials which can be used according to the invention are: geraniol, geranyl acetate, linalool, linalyl acetate, tetrahydrolinalool, citronellol, citronellyl acetate, dihydromyrcenol, dihydromyrcenyl acetate, tetrahydromyrcenol, terpineol, terpinyl acetate, nopol, nopyl acetate, 2-phenyl-ethanol, 2-phenyl-ethyl acetate, benzyl alcohol, benzyl acetate, benzyl salicylate, styrallyl acetate, benzyl benzoate, amyl salicylate, dimethylbenzyl-carbinyl acetate, trichloro-methylphenyl-carbinyl acetate, p-tert-butylcyclohexyl acetate, isononyl acetate, vetiveryl acetate, vetiverol, αhexylcinnamaldehyde, 2-methyl- 3-(p-tert-butylphenyl) propanal, 2-methyl-3-(p-isopropylphenyl)propanal, 2-(p-tert-butylphenyl)-propanal, 2,4-dimethyl-cyclohex-3-enylcarboxaldehyde, tricyclodecenyl acetate, tricyclodecenyl propionate, 4-(4-hydroxy-4methylpentyl)-3-cyclohexenecarboxyaldehyde, 4-(4-methyl-3-pentenyl)-3-cyclohexenecarboxaldehyde, 4-acetoxy-3-pentyltetrahydropyran, 3-carboxy methyl-2-pentylcyclopentanone, 2-n-heptylcyclopentanone, 3-methyl-2-pentyl-2-cyclopentenone, n-decanal, n-dodecanal, 9-decenol-1, phenoxyethylisobutyrate, phenylacetaldehydedimethylacetal, phenylacetaldehyde diethyl acetal, geranyl nitrile, citronellyl nitrile, cedryl acetate, 3isocamphylcyclohexanol, cedryl methyl ether, isolongifolanone, aubepine nitrile, anisic aldehyde, heliotropin, coumarin, eugenol, vanillin, diphenyl oxide, hydroxycitronellal, ionones, methylionones, isomethylionones, irones, cis-3-hexenol and esters thereof, indanemusks, tetralinmusks, isochromanmusks, macrocyclic ketones, macrocyclic lactone musks, ethylene brassylate.

Other fragrances are also possible according to International Cosmetic Ingredient Dictionary and Handbook, 12th ed. 2008, Vol. 3, p. 3193-3200 (edited by CTFA, Washington DC, USA).

In a preferred embodiment of the invention the fragrance is fragrance oil. A fragrance or fragrance oil in the context of the present invention comprises at least one volatile component i.e. a sensory active substance. Fragrances typically are complex mixtures of volatile substances which can be extracts of natural raw materials such as for example ambergris tincture; amyris oil; angelica seed oil; angelica root oil; aniseed oil; valerian oil; basil oil; wood moss absolute; bay oil; mugwort oil; benzoin resin; bergamot oil; beeswax absolute; birch tar oil; bitter almond oil; savory oil; bucco-leaf oil; cabreuva oil; cade oil; calamus oil; camphor oil; cananga oil; cardamom oil; cascarilla oil; cassia oil; cassia absolute; castoreum absolute; cedar-leaf oil; cedarwood oil; cistus oil; citronella oil; lemon oil; copaiba balsam; copaiba balsam oil; coriander oil; costus root oil; cumin oil; cypress oil; davana oil; dill oil; dillseed oil; eau de brouts absolute; oakmoss absolute; elemi oil; tarragon oil; eucalyptus citriodora oil; eucalyptus oil; fennel oil; fir oil; galbanum oil; galbanum resin; geranium oil; grapefruit oil; guaiac wood oil; gurjun balsam; gurjun balsam oil; helichrysum absolute; helichrysum oil; ginger oil; iris root abolute; iris root oil; jasmine absolute; calamus oil; blue camomile oil; Roman camomile oil; carrotseed oil; cascarilla oil; pine-needle oil; spearmint oil; caraway oil; labdanum oil; labdanum absolute; labdanum resin; lavandin absolute; lavandin oil; lavender absolute; lavender oil; lemongrass oil; lovage oil; distilled lime oil; pressed lime oil; linaloe oil; litseacubeba oil; bay-leaf oil; mace oil; marjoram oil; mandarin oil; massoi bark oil; mimosa absolute; ambrette oil; tincture of musk; clary sage oil; myristica oil; myrrh absolute; myrrh oil; myrtle oil; clove leaf oil; clove flower oil; neroli oil; olibanumabolute; olibanum oil; opopanax oil; orange blossom absolute; orange oil; origanum oil; palmarosa oil; patchouli oil; perilla oil; Peru balsam oil; parsley leaf oil; parsley seed oil; petitgrain oil; peppermint oil; pepper oil; pimento oil; pine oil; pennyroyal oil; rose absolute; rosewood oil; rose oil; rosemary oil; Dalmatian sage oil; Spanish sage oil; sandalwood oil; celery seed oil; spike lavender oil; Japanese aniseed oil; styrax oil; tagetes oil; fir-needle oil; teatree oil; turpentine oil; thyme oil; Tolu balsam; tonka absolute; tuberose absolute; vanilla extract; violet leaf absolute; verbena oil; vetiver oil; juniper oil; wine-lees oil; wormwood oil; wintergreen oil; ylang oil; hyssop oil; civet absolute; cinnamon leaf oil; cinnamon bark oil; as well as fractions thereof or constituents isolated therefrom.

Individual volatile fragrance substances are for example hydrocarbons, aliphatic alcohols, alphatic aldehydes and their acetals, aliphatic ketones and oximes thereof, aliphatic sulphur-containing compounds, aliphatic nitriles, aliphatic carboxylic acids and esters thereof, acyclic terpene alcohols, acyclic terpene aldehydes and ketones, cyclic terpene alcohols, cyclic terpene aldehydes and ketones, cyclic alcohols, cycloaliphatic alcohols, cyclic and cycloaliphatic ethers, cyclic ketones, cycloaliphatic aldehydes, cycloaliphatic ketones, esters of cyclic alcohols, esters of cycloaliphatic carboxylic acids, aromatic hydrocarbons, araliphatic alcohols, esters of araliphatic alcohols and aliphatic carboxylic acids, aromatic and araliphatic aldehydes, aromatic and araliphatic ketones, aromatic and araliphatic carboxylic acids and esters thereof, nitrogen-containing aromatic compounds, phenols, heterocyclic compounds and lactones.

The fragrances and fragrance-fixing complexes described in US 2013/0209385 A1 can also be used in the present invention.

According to the present invention, the fragrance is contained in the biphasic composition in an amount of 1 to 50 wt%, preferably in an amount of 2 to 30 wt% and more preferred in an amount of 5 to 20 wt% based on the total weight of the composition.

The definition of the expression "second fragrance" as used in the present invention is the same as for "first fragrance" but with the provision that within the particular embodiments, the second fragrance is not the same compound as the first fragrance. Preferably, the second fragrance is diverse from the first fragrance. This means that if the first fragrance is e.g. hexylcinnamic aldehyde the second fragrance can be any fragrance except hexylcinnamic aldehyde.

In a particular preferred embodiment, the first fragrance and the second fragrance are predominantly in different phases of the composition. This means that the first fragrance is either in the upper phase of the biphasic composition and the second fragrance is in the bottom phase of the biphasic composition or vice versa.

Whether the first fragrance or second fragrance disperses rather in the aqueous or in the oily phase of the biphasic composition depends on their solubility in these two phases and/or on their partition coefficient.

The way how a certain compound dispenses in the aqueous or in the oily phase also depends on the percentage of non-polar component(s) within the oily phase of the biphasic composition of the invention. Surprisingly, it has been found that by regulating the amount of non-polar component(s) in the oily phase it is possible to achieve that a defined amount of a compound dispenses in a particular phase. The non-polar component(s) which can be added to the oily phase preferably have a log P-value $\geq 10$. Examples for such ingredients are ethylhexyl palmitate or pentaerythrityl tetraoleate.

In a particular preferred embodiment, more than 50 wt% of the first fragrance is contained in one of the two phases of the composition based on the total weight of the first fragrance. Thus, more than 50 wt% of the first fragrance is located e.g. in the upper phase and less than 50 wt% of the first fragrance is located in the bottom phase of the biphasic composition or vice versa.

In a particular preferred embodiment, at least 60 wt% of the first fragrance is contained in one of the two phases of the composition based on the total weight of the first fragrance. Therefore, at least 60 wt% of the first fragrance is located e.g. in the upper phase and maximum 40 wt% of the first fragrance is located in the bottom phase of the biphasic composition or vice versa.

In a particular preferred embodiment, at least 70 wt% of the first fragrance is contained in one of the two phases of the composition based on the total weight of the first fragrance. This means that at least 70 wt% of the first fragrance is located e.g. in the upper phase and maximum 30 wt% of the first fragrance is located in the bottom phase of the biphasic composition or vice versa.

In a particular preferred embodiment, more than 50 wt% of the second fragrance is contained in one of the two phases of the composition based on the total weight of the second fragrance. Hence, more than 50 wt% of the second fragrance is located e.g. in the upper phase and less than 50 wt% of the second fragrance is located in the bottom phase of the biphasic composition or vice versa.

In a particular preferred embodiment, at least 60 wt% of the second fragrance is contained in one of the two phases of the composition based on the total weight of the second fragrance. This means that at least 60 wt% of the second fragrance is located e.g. in the upper phase and maximum 40 wt% of the second fragrance is located in the bottom phase of the biphasic composition or vice versa.

In a particular preferred embodiment, at least 70 wt% of the second fragrance is contained in one of the two phases of the composition based on the total weight of the second fragrance. This means that at least 70 wt% of the second fragrance is located e.g. in the upper phase and maximum 30 wt% of the second fragrance is located in the bottom phase of the biphasic composition or vice versa.

Each phase of the biphasic composition may comprise predominantly a first fragrance or second fragrance. Each phase of the biphasic composition may also comprise blends of the first and the second fragrance wherein both can be present in varying amounts. This can be achieved by adjusting the amount of the polar compound in the water/alcohol phase. Alternatively or additionally this can also be achieved by adjusting the amount of non-polar components in the oily phase. If one of the two phases predominantly comprises either the first fragrance or the second fragrance this means that both phases of the biphasic composition smell differently. In other words, one phase can for example have a lemon-like scent and the other phase can smell like lavender. Upon agitation prior to use of the cosmetic composition, the first fragrance and the second fragrance are mixed up and a combined new odor is produced which can then be distributed on the body or clothes etc. When agitating vigorously, both phases can form an emulsion-like fluid.

The combined new odor can be characterized as a "third fragrance". According to the present invention, the definition of the expression "third fragrance" is the same as for "first and/or second fragrance" but with the provision that within the particular embodiments, the "third fragrance" presents an odor that is different from the individual odors of the "first and/or second fragrance".

The biphasic composition of the present invention contains one or more branched hydrocarbons. According to the present invention, the biphasic composition comprises or consists of alcohol, water, a fragrance and a hydrocarbon and does not comprise volatile linear alkanes. The term "hydrocarbon" in the expression "branched hydrocarbon" is intended to mean an organic compound consisting entirely of hydrogen and carbon. "Branched" in the expression "branched hydrocarbon" means that the carbon backbone diverges in one or more directions, i.e. it is not straight or linear. To distinguish branched from linear hydrocarbons the prefix "iso-" is used, e.g. isododecane.

The skilled person knows branched hydrocarbon that are suitable for the present invention. Also mixtures of different branched hydrocarbons or mixtures of branched and linear hydrocarbons are possible. In general, branched hydrocarbons are soluble in alcohol e.g. in ethanol, whereas linear hydrocarbons are not.

Preferably, the branched hydrocarbon(s) comprises 5 to 20 carbon atoms, more preferably 8 to 19 and even more preferred 9 to 16 carbon atoms.

According to the present invention, the branched hydrocarbon is contained in the biphasic composition in an amount of 5 to 60 wt%, preferably in an amount of 10 to 40 wt% and more preferred in an amount of 15 to 35 wt% based on the total weight of the composition.

Preferably, the branched hydrocarbon is an alkane. The term "alkane" means a saturated hydrocarbon which is composed entirely of single bonds. Examples for alkanes that are suitable for use the present invention are isopentane ($C_5$), isohexane ($C_6$), isoheptane ($C_7$), isooctane ($C_8$), isononane ($C_9$), isodecane ($C_{10}$), isoundecane ($C_{11}$), isododecane ($C_{12}$), isotridecane ($C_{13}$), isotertadecane ($C_{14}$), isopentadecane ($C_{15}$), isohexadecane ($C_{16}$), isoheptadecane ($C_{17}$), isooctadecane ($C_{18}$), isononadecane ($C_{19}$) and isoeicosane ($C_{20}$). Preferably, the alkane is isododecane.

According to the present invention, the biphasic composition may comprise cosmetically acceptable auxiliary substances. The person skilled in the art is aware which cosmetically acceptable auxiliary substances can be used for the present invention. Preferably, colorants, skin conditioning agents, humectants, emollients, wetting agents, silicon oil or gum, natural or synthetic oils, powders, fillers, opacifying agents, preservatives, flavoring agents or mixtures thereof are used as cosmetically acceptable auxiliary substances.

The biphasic composition of the present invention may comprise no colorant so that both phases of the biphasic composition are colorless. Alternatively, the biphasic composition of the present invention may comprise a colorant. According to the present invention, the colorant may comprise at least a first colorant and a second colorant.

The term "colorant" or "first colorant" as used in the present invention means a substance (e.g. a dye or pigment) that colors something. The colorant can comprise a mixture of two or more coloring substances. As colorants cosmetically acceptable pigments, natural or synthetic organic dyes or natural or synthetic inorganic dyes and mixtures thereof can be used. Examples for pigments which can be used are made of titanium oxide, titanium dioxide, stainless steel, silica, tin oxide, sodium and/or alcium and/or aluminium borosilicate, polyester glitter, zirconium oxide or cerium oxide, and also zinc oxide, iron (II; III) oxide, such as, Brown Iron Oxide C33-115, Yellow Iron Oxide CG 450, Yellow Iron Oxide C33-1700, Red Iron Oxide C33-2199 or Black Iron Oxide C33-5000, chromium oxide, such as Chrome Oxide Green CG 525 or bismuth oxychloride. Further, ferric blue, manganese violet, copper powder and bronze powder can be used. As organic pigments the use of carbon black, and barium, strontium, calcium (D&C Red No. 7) and aluminium lakes is possible. Also nacres or goniochromatic pigments, for example multilayer interference pigments, and/or reflective pigments may be included into the biphasic composition. As nacres coated mica can be used. The mica can be of natural or synthetic origin e.g. synthetic fluorphlogopite. The mica can be coated with metal oxides like titanium oxide, iron oxide, natural pigments, bismuth oxychloride or other typical cosmetic ingredients. Dyes may be soluble in the aqueous or the oily phase of the biphasic composition. Suitable oil-soluble dyes are for example D&C Orange 5, D&C Red 17, D&C Green 6, D&C Yellow 11, D&C Violet 2, Annatto, Lycopene, Turmeric, Beta-carotene, Phat Orange DC 2204, Phat Blue DC 6204, Phat Green DC 7215, Phat Brown DC 8206, Phat Black DC 9203, Phat Black DC 9206. Suitable water-soluble dyes are for example FD&C Yellow 5, FD&C Yellow 6 ,FD&C Yellow 8, FD&C Yellow 10, FD&C Ext-Yellow 7, FD&C Orange 4, , FD&C Red 4, FD&C Red 40, FD&C Red 22, FD&C Red 28, D&C Red 33, Ext-DC Violet 2, FD&C Blue 1, FD&C Green 3, FD&C Green 5, FD&C Green 8, D&C Brown 1, quinoline yellow and beetroot juice.

According to the present invention, the colorant is contained in the biphasic composition in an amount of 0.0000001 to 1 wt%, preferably in an amount of 0.00001 to 0.5 wt% and more preferably in an amount of 0.001 to 0.4 wt% based on the total weight of the composition.

According to the present invention, the colorant of the biphasic composition may comprise no second colorant. In a preferred embodiment, the first colorant is in one of the two phases of the biphasic composition and the other phase does not comprise a second colorant. Therefore, both phases differ in their appearance, since one phase is colored with the first colorant and the other phase is colorless since it does not comprise a second colorant. Upon agitation prior to use of the biphasic composition, the colored phase and the colorless phase are mixed up and a new color blend is produced. This third color may e.g. be a light color version of the colorant which was in one of the phases.

The definition of the expression "second colorant" as used in the present invention is the same as for "first colorant" but with the provision that within the particular embodiments, the second colorant is not the same compound as the first colorant. Preferably, the second colorant is diverse from the first colorant. This means that if the first colorant gives e.g. a green color, the second colorant can be any coloring substance except the one that gives the green color as the first colorant.

In a particular preferred embodiment, the first colorant and the second colorant are predominantly in different phases of the composition. This means that the first colorant is either in the upper phase of the biphasic composition and the second colorant is in the bottom phase of the biphasic composition or vice versa. In other words, one phase can e.g. be colored red and the other phase can be yellow.

The way how the first colorant and the second colorant dispense in the aqueous or in the oily phase of the biphasic composition depends on their solubility in these two phases as well as on their log P-value. For example "D&C Yellow No. 11" is oil-soluble, whereas "FD&C Yellow No.5" is water-soluble.

In a particular preferred embodiment, more than 50 wt% of the first colorant is contained in one of the two phases of the composition based on the total weight of the first colorant. Thus, more than 50 wt% of the first colorant is located e.g. in the upper phase and less than 50 wt% of the first colorant is located in the bottom phase of the biphasic composition or vice versa.

In a particular preferred embodiment, at least 60 wt% of the first colorant is contained in one of the two phases of the composition based on the total weight of the first colorant. Therefore, at least 60 wt% of the first colorant is located e.g. in the upper phase and maximum 40 wt% of the first colorant is located in the bottom phase of the biphasic composition or vice versa.

In a particular preferred embodiment, at least 70 wt% of the first colorant is contained in one of the two phases of the composition based on the total weight of the first colorant. This means that at least 70 wt% of the first colorant is located e.g. in the upper phase and maximum 30 wt% of the first colorant is located in the bottom phase of the biphasic composition or vice versa.

In a particular preferred embodiment, more than 50 wt% of the second colorant is contained in one of the two phases of the composition based on the total weight of the second colorant. Hence, more than 50 wt% of the second colorant is located e.g. in the upper phase and less than 50 wt% of the second colorant is located in the bottom phase of the biphasic composition or vice versa.

In a particular preferred embodiment, at least 60 wt% of the second colorant is contained in one of the two phases of the composition based on the total weight of second colorant. This means that at least 60 wt% of the second colorant is located e.g. in the upper phase and maximum 40 wt% of the second colorant is located in the bottom phase of the biphasic composition or vice versa.

In a particular preferred embodiment, at least 70 wt% of the second colorant is contained in one of the two phases of the composition based on the total weight of the second colorant. This means that at least 70 wt% of the second colorant is located e.g. in the upper phase and maximum 30 wt% of the second colorant is located in the bottom phase of the biphasic composition or vice versa.

Each phase of the biphasic composition may comprise predominantly a first colorant or second colorant. Each phase of the biphasic composition may also comprise blends of the first and the second colorant wherein both can be present in varying amounts. If one of the two phases predominantly comprises either the first colorant or the second colorant this means that both phases of the biphasic composition appear in a different color. On agitation prior to use of the cosmetic composition, the first colorant and the second colorant are mixed up and a new color blend is produced. This combined new color blend can be characterized as a "third color". According to the present invention, the definition of the expression "third color" is the same as for "first and/or second color" but with the provision that within the particular embodiments, the "third color" presents a color that is different from the individual colors of the "first and/or second color".

Also skin conditioning agents may be used as cosmetically acceptable auxiliary substances. Suitable skin conditioning agents are cosmetic ingredients which help to maintain the soft, smooth and pliable appearance of the skin and can also act as humectants, emollients or occlusives. Skin conditioning agents help to strengthen the skins barrier function and act by attracting and holding moisture in the skin.

Suitable skin conditioning agents are known to the skilled person. Examples are vitamins, mineral salts, trace elements, plant extracts, animal extracts, proteins or enzymes, all type of film former ingredients, all type of ingredient having a cationic charge, conditioning silicon oils, natural and/or synthetic oils, and/or fats, allantoin, L-carnitine, extracts of plants of the genus aloe, preferably aloe vera, aloe barbadensis or aloe andongensis, chamomile essence, ceramides, cholesterol, acrylic polymers, cellulosic polymers, phenyl trimethicone or compounds containing polyethylene glycol and/or polypropylene glycol.

Every natural or synthetic oil which is used in the personal care/cosmetic industry is suitable for the biphasic composition according to the invention. In particular, saturated and/or unsaturated vegetable oils, for instance nut oils, such as *Aleurites Moluccana* nut oil, brazil nut oil, groundnut oil, hazel nut oil, macadamia nut oil, peanut oil,apricot oil, avocado oil, borage oil, butyl stearate, $C_{12}$-$C_{15}$ alkyl benzoates, $C_{10}$ - $C_{20}$ chain triglycerides, cacao butter, calendula oil, castor oil, cetyl alcohols, cetylpalmitate, coconut oil, cotton seed oil, evening primrose oil, grape seed oil, jojoba oil, mango butter, medium chain ($C_6$ to $C_{12}$) triglycerides (MCTs), mineral oil, mink oil, olive oil, palm kernel oil, rapeseed oil, retinal oil, ricinus oil, safflower oil, sesame oil, shea butter, soy bean oil, spermaceti oil, St. John's wort oil, sun flower oil, sweet almond oil, sweet corn oil, thistle oil or wheat germ oil and mixtures thereof are preferably used. According to the invention, isopropyl myristate is preferred as natural/synthetic oil.

Suitable humectants that may be used in the present invention are known to the skilled person. Examples for wetting agents are sorbitol, polyethylene glycols like Lutrol E400 (e.g. available from BASF), various glycols like polyglycol, butyl glycol and/or glycol polyols like Zemea (e.g. available from Dupont)or Hydrolite 5 (e.g. available from Symrise), glycerin and derivatives thereof, for instance polyglycerin 10, decaglyceryldecaoleatesorbitantrioleate, polyglycerol-3-diisostearate, sorbitansesquioleate, glycerol triisostearate, diethanolamine or lecithin. In a preferred embodiment the biphasic composition of the invention contains lecithin as a wetting agent, for example Emulmetik 100 (e.g. available from Lukas Meyer, Germany).

According to the invention, emollients can be added to prevent water lost and to soften and smoothen the skin. Suitable emollients are for instance silicones, such as Trisiloxane, Disiloxane, cyclomethicone (Hexa, Penta), dimethicone, alkyl dimethicone or alkyl silicone (e.g. Dow Corning 2503 Cosmetic wax and Dow Corning AMS C-30 Wax), hydrocarbons, fatty alcohols, synthetic and natural oils, petrolatum based emollients, such as mineral oil, petroleum jelly or lanolin. Other suitable emollients are known by the skilled person.

The biphasic composition may further comprise opacifying agents that make the composition milky or cloudy. Suitable opacifying agents are for instance treated or untreated titanium dioxide, Ethalkonium Chloride, Acrylate/HEMA/Styrene Copolymer, Styrene/Acrylates Copolymer and Acrylates/C12-22 Alkyl Methacrylate Copolymer.

The biphasic composition may further comprise preservatives to stabilize the composition over time. Suitable preservatives are for instance parabens, phenoxyethanol, caprylyl glycol, sorbic acid, glucono delta lactone, sodium benzoate or any typical preservative or mixtures thereof used in the personal care/cosmetic industry.

Cosmetically acceptable fillers are known by the skilled person. Suitable fillers are for example mineral silicates, such as mica and talc, starch, kaolin, nylon, zinc oxide, titanium oxide, precipitated calcium carbonate or synthetic polymer powders, such as Silicon crosspolymer powder, acrylate powders, for instance polymethylmetacrylate (PMMA) powders and combinations thereof.

The biphasic composition of the invention may comprise flavoring agents or ingredients to enhance the taste of the cosmetic product. Cosmetically acceptable flavoring agents or ingredients are known to the skilled person. The flavoring agents or ingredients may be of natural origin, such as plant and fruit extracts or essences. The flavoring agents or ingredients may also be of synthetic origin.

According to the present invention, the biphasic composition may comprise additional cosmetically active substances. The person skilled in the art is knows additional cosmetically active substances. Preferably, one or more of the additional cosmetically active substances are predominantly in one phase of the biphasic composition. Preferably, anti-oxidants, anti-aging agents, anti-wrinkles agents, sun protectants, cooling actives, insect repellents or mixtures thereof are used as additional cosmetically active substances.

Suitable antioxidants are for instance vitamins, such as vitamin C and derivatives thereof, for instance ascorbyl acetate, ascorbyl phosphate, ascorbylpalmitate or magnesium ascorbyl phosphate, vitamin A and derivatives thereof, folic acid and derivatives thereof, vitamin E and derivatives thereof, such as tocopheryl acetate, flavones and flavonoides, amino acids, such as histidine, glycine, tyrosine, tryptophan and derivatives thereof, imidazoles, such as cis- or trans-urocanic acid and derivatives thereof, peptides, such as D,L-carnosine, D-carnosine, L-carnosine and derivatives thereof, carotenoides, such as xanthophylles and carotenes, such as $\alpha$-carotene, ß-carotene, lycopine, uric acid and derivatives thereof, $\alpha$ hydroxy acids, such as citric acid, lactic acid, malic acid, $\alpha$ hydroxy fatty acids, such as palmitic acid, phytic acid, lactoferrin, stilbenes and derivatives thereof, mannose and derivatives thereof, liponic acid and derivatives thereof, such as dihydrolipoic acid, ferulic acid and derivatives thereof, thiols, such as glutathion, cysteine, cystine and esters thereof, folic acid and derivatives thereof or polyphenoles and derivatives thereof.

Anti-oxidants are also the most powerful anti-aging and anti-wrinkles agents. According to the invention further all substances which tight the skin or accelerate healing, repairing or renewing processes can be included as anti-aging and anti-wrinkles agents into the biphasic composition of the invention. Suitable examples are plant extracts, coenzymes and enzymes, provitamins, trace elements, hyaluronic acid, proteins and peptides, in particular proteins of the extracellular matrix or peeling agents.

UV-filter can be added to the biphasic composition of the invention to protect the skin from UVA and UVB radiation. Suitable oil soluble UVB-filters are 4-aminobenzoic acid and derivatives thereof, such as octyl dimethyl para-aminobezoic acid or 4-dimethylaminobenzoic acid-2-ethylhexyl ester, cinnamic acid and derivatives thereof, such as octyl p-methoxycinnamate or 4-methoxycinnamic acid-2-ethylhexyl ester, benzophenones and derivatives thereof, such as 2-hydroxy-4-methoxybenzophenone or benzophenone-3, 3-benzylidene camphor and derivatives thereof, such as methylbenzylidene camphor, butyl methoxydibenzoylmethane, octyl salicylate, or homosalate and mixtures thereof.

Suitable water soluble UVB-filters are sulfonic acid derivatives of benzophenone or 3-benzylidene camphor or salts thereof, such as sodium or potassium of 2-phenylbenzimidazole-5- sulfonic acid.

Suitable UVA-filters are dibenzoylmethane and derivatives thereof, such as 1-phenyl-4-(4'-isopropylphenyl)propane-1,3-dione, butyl methoxydibenzoylmethane or menthylanthranilate or mixtures thereof. According to the invention broad band-pass filter can also be used, such as derivatives of bis-resorcinyltriazine or benzoaxoles.

As insect repellent, plant extracts, such as citric oil, orange oil, melissa oil, eucalyptus oil, lavender oil or pink oil or diethyl toluamide or mixtures thereof are used. Suitable other insect repellents are known to the skilled person.

To impart a fresh and comfortable feeling to the skin cooling actives can be formulated into the biphasic composition of the invention. Suitable cooling actives according to the invention are menthol and its derivatives, for instance menthyl lactate (Frescolat ML) or menthone glycerin acetal (Frescolat MGA).

Each phase of the biphasic composition may comprise predominantly one or more cosmetically active substances or one or more cosmetically acceptable auxiliary substances. This means that one phase can for example contain an anti-oxidant and the other phase can contain a humectant. This can be achieved by choosing substances with appropriate solubility character and/or log P-values. Each phase of the biphasic composition may also comprise blends of cosmetically active substances and cosmetically acceptable auxiliary substances wherein both can be present in varying amounts. Upon agitation prior to use of the biphasic composition, the cosmetically active substance(s) and the cosmetically acceptable auxiliary substance(s) are mixed up and a combined new composition is produced which can then be distributed on the body or clothes etc.

According to the present invention, the biphasic composition may comprise one or more types of solid particles. The person skilled in the art knows appropriate solid particles. The solid particles can be present at the bottom of one of the phases of the composition, on top of one of the phases of the composition or floating within one phase or within both phases of the composition. The solid particles can be of any suitable size, any color and can be loaded with a gas. The solid particles can be of any porosity and can be made e.g. of polypropylene (PP), polyethylene (PE), PCTA, TPX, Surlyn, Silicon (Xiameter RTV), acrylic Crosslinked copolymer superabsorbent.

According to the present invention, the biphasic composition may comprise one or more gelling agents to give the biphasic composition a certain aesthetic appearance. The gelling agents can be in the water-alcohol phase, the oily phase or in both phases of the biphasic composition. Examples for suitable gelling agents are e.g. Polyacrylate Crosspolymer-6, Polyacrylate Crosspolymer-11, Carbomer and Ammonium Acryloyldimethyltaurate-Carboxyethyl Acrylate-crosspolymer. Compatible hydro-alcoholic gelling ingredients have been described in WO2013/012939 A1.

In a preferred embodiment, the biphasic composition comprises or consists of:

20 - 75 wt% alcohol;
0.5 - 30 wt% polar compound;
1 - 50 wt% fragrance and
5 - 60 wt% branched hydrocarbon.

In another preferred embodiment, the biphasic composition comprises or consists of:

40 - 65 wt% alcohol;
2 - 9 wt% polar compound;
5 - 20 wt% fragrance and
15 - 35 wt% branched hydrocarbon.

In another preferred embodiment, the biphasic composition comprises or consists of:

40 - 65 wt% alcohol;
2 - 9 wt% polar compound;
5 - 20 wt% fragrance;
15 - 35 wt% branched hydrocarbon; and
15 - 35 wt% linear hydrocarbon.

In a particular preferred embodiment, the biphasic composition comprises or consists of:

40 - 65 wt% ethanol;
2 - 9 wt% water;
5 - 20 wt% Fragrance Oil 5420492 and
15 - 35 wt% isododecane.

In a particular preferred embodiment, the biphasic composition comprises or consists of:

40 - 65 wt% ethanol;
2 - 9 wt% water;
5 - 20 wt% Fragrance Oil 173250 and
15 - 35 wt% Isopar L.

In a particular preferred embodiment, the biphasic composition comprises or consists of:

40 - 65 wt% ethanol;
2 - 9 wt% water;
5 - 20 wt% Fragrance Oil 173250 and
15 - 35 wt% Isopar M.

In a particular preferred embodiment, the biphasic composition comprises or consists of:

40 - 65 wt% ethanol;
2 - 9 wt% water;
5 - 20 wt% Fragrance Oil 173250 and
15 - 35 wt% Isopar N.

In a particular preferred embodiment, the biphasic composition comprises or consists of:

40 - 65 wt% ethanol;
2 - 9 wt% water;
5 - 20 wt% Fragrance Oil 173250 and
15 - 35 wt% Isopar V.

In a particular preferred embodiment, the biphasic composition comprises or consists of:

40 - 65 wt% ethanol;
2 - 9 wt% water;
5 - 20 wt% Fragrance Oil 5420492 and
15 - 35 wt% isohexadecane.

In a particular preferred embodiment, the biphasic composition comprises or consists of:

40 - 65 wt% ethanol;
2 - 9 wt% water;
5 - 20 wt% Fragrance Oil 5420492 and
15 - 35 wt% isoeicosane.

In a particular preferred embodiment, the biphasic composition comprises or consists of:

40 - 65 wt% ethanol;
2 - 9 wt% water;
5 - 20 wt% Fragrance Oil 5420492;
15 - 35 wt% isododecane; and
15 - 35 wt% lilac.

The present invention is also directed to the use of the biphasic composition for fragrance applications. Such applications mean using the fragrance of the present invention for perfuming the body, hair, clothes or the surroundings.
Preferably, the biphasic composition is an eau fraîche, eau de toilette, eau de parfum, perfume, fragrance elixir, perfume oil, after shave or deodorant. More preferably, the biphasic composition is an eau de toilette.
The present invention has several advantages over the state of the art: The biphasic composition represents a new product with two visible phases. These phases can both be colored, preferably by different colorants. It is also possible that only one of the phases is colored and the other phase is colorless. Due to the visibility of the distinct phases it is possible to use this composition with or without shaking it. Since the phases comprise different fragrances, the consumer can decide whether he wants to use only one of the fragrances separately or a mixture of the fragrances. He also has the option to mix both phases completely or only partially to obtain a novel mixing ratio and therefore a new scent. The biphasic composition of the present invention makes it therefore possible to dispense at least three different scents by using each of the two phases alone or a mixture of both phases in combination. It is also possible to use an adapted packaging device to spray only the upper or only the lower phase or the combination of both phases.

The biphasic composition of the present invention shall be exemplified by the following figures and examples.

FIGURES

Figure 1: Study on single fragrance migration in different biphasic compositions.

Figure 2: Stability of individual fragrances in the phases of the biphasic composition.

EXAMPLES

The constituents of the oily phase those of the aqueous phase were mixed. Then the two phases were mixed together. It is also possible to mix all constituents directly together in order to reach the biphase equilibrium phase quickly.

The composition obtained, when left to stand, comprised a distinct colored transparent oily phase and a distinct colored transparent aqueous phase. Upon agitation, the two phases produced an opaque composition. In most of the case the oil phase will form the top phase of the composition due to its lower density and the higher density water-alcohol phase will form the bottom phase of the composition.

According to the present invention, the alcohol is preferably perfumery grade alcohol. The alcohol may comprise up to 10% water, preferably from 5% to 8%water. The alcohol may be pure or may comprise one or more denaturing agents suitable for this use known by the skilled person. Preferably, ethanol or other short carbon chain alcohols like e.g. isopropyl alcohol can be used.

The total water content is calculated on the amount of water within the alcohol and the water amount that is comprised in the composition.

The resulting compositions were all biphasic except where stated otherwise.

**Example 1:**

|  | wt% |
| --- | --- |
| Isododecane | 29% |
| Isopropyl myristate | 3.125% |
| DC Yellow 11 solution 0.2% in IPM | 0.125% |
| Fragrance oil | 12% |
| Alcohol SDA 40B 190P | 49% |
| UV blend | 2% |
| water, deionized | 4.73% |
| FDC Yellow #5 0.2% solution in water | 0.02% |
|  | (total water content ∼8.5 wt%) |

"Alcohol SDA 40B 190P" corresponds to an alcohol-water mixture with ∼92.2% pure ethanol and ∼7.8% DI water.

**Example 2:**

|  | Wt% |
| --- | --- |
| ISOPAR L | 29 |
| IMP | 10.23 |
| YELLOW LIPO 0.1%IMP | 0.45 |

(continued)

| | Wt% |
|---|---|
| Fragrance Oil 173250 | 6 |
| ALCOHOL 96.2% | 47.8 |
| UV BLEND | 2 |
| WATER | 4.5 |
| RED 33 1% | 0.02 |
| | (total water content ~7.2 wt%) |

"Alcohol 96.2%" corresponds to an alcohol-water mixture with ~94.2% pure ethanol and ~5.7% DI water.

**Example 3:**

| | wt% |
|---|---|
| ISOPAR M | 29 |
| IMP | 10.23 |
| YELLOW LIPO 0.1%IMP | 0.45 |
| Fragrance Oil 173250 | 6 |
| ALCOHOL 96.2% | 47.8 |
| UV BLEND | 2 |
| WATER | 4.5 |
| RED 33 1% | 0.02 |
| | (total water content ~7.2 wt%) |

**Example 4:**

| | wt% |
|---|---|
| ISOPAR N | 29 |
| IMP | 10.7 |
| Fragrance Oil 173250 | 6 |
| ALCOHOL 96.2% | 47.8 |
| UV BLEND | 2 |
| WATER | 4.5 |
| | (total water content ~7.2 wt%) |

**Example 5:**

| | wt% |
|---|---|
| ISOPAR V | 29 |
| IMP | 10.7 |
| Fragrance Oil 173250 | 6 |
| ALCOHOL 96.2% | 47.8 |
| UV BLEND | 2 |
| WATER | 4.5 |
| | (total water content ~7.2 wt%) |

**Example 6:**

|  | wt% |
| --- | --- |
| PERMETHYL 101 A | 29 |
| IMP | 10.7 |
| Fragrance Oil 5420492 | 6 |
| ALCOHOL 96.2% | 47.8 |
| UV BLEND | 2 |
| WATER | 4.5 |
|  | (total water content ~7.2 wt%) |

**Example 7:**

|  | wt% |
| --- | --- |
| PERMETHYL 102 A | 29 |
| IMP | 10.7 |
| Fragrance Oil 5420492 | 6 |
| ALCOHOL 96.2% | 47.8 |
| UV BLEND | 2 |
| WATER | 4.5 |
|  | (total water content ~7.2 wt%) |

**Example 8:**

|  | wt% |
| --- | --- |
| ISODODECANE | 19.87 |
| LILAC | 19.87 |
| Fragrance Oil 5420492 | 6 |
| UV BLEND | 2 |
| ALCOHOL 96.2% | 52.3 |
|  | (total water content ~3.0 wt%) |

**Example 9:**

|  | wt% |
| --- | --- |
| ISODODECANE | 19.87 |
| LILAC | 19.87 |
| Fragrance Oil 5420492 | 6 |
| UV BLEND | 2 |
| ALCOHOL 96.2% | 47.8 |
| WATER | 4.5 |
|  | (total water content ~7.2 wt%) |

**Example 10:**

|  | wt% |
|---|---|
| ISODODECANE | 29 |
| IMP | 8.7 |
| EUMULGIN L | 2 |
| Fragrance Oil 5420492 | 6 |
| ALCOHOL 96.2% | 47.8 |
| UV BLEND | 2 |
| WATER | 4.5 |
|  | (total water content ∼7.2 wt%) |

**Example 11:**

|  | wt% |
|---|---|
| ISODODECANE | 29 |
| IMP | 8.7 |
| CREMOPHOR CO 40 | 2 |
| Fragrance Oil 5420492 | 6 |
| ALCOHOL 96.2% | 47.8 |
| UV BLEND | 2 |
| WATER | 4.5 |
|  | (total water content ∼7.2 wt%) |

**Example 12:**

|  | wt% |
|---|---|
| ISODODECANE | 29 |
| IMP | 8.7 |
| PLANTACARE 2000 | 2 |
| Fragrance Oil 5420492 | 6 |
| ALCOHOL 96.2% | 47.8 |
| UV BLEND | 2 |
| WATER | 4.5 |
|  | (total water content ∼7.2 wt%) |

**Example 13:**

|  | wt% |
|---|---|
| ISODODECANE | 29 |
| IMP | 8.7 |
| TEXAPON N25 BZ | 2 |
| Fragrance Oil 5420492 | 6 |
| ALCOHOL 96.2% | 47.8 |
| UV BLEND | 2 |

(continued)

|  | wt% |
|---|---|
| WATER | 4.5 |
| | (total water content ∼7.2 wt%) |

**Example 14:**

|  | wt% |
|---|---|
| ISODODECANE | 29 |
| IMP | 8.7 |
| TEGO F 50 | 2 |
| Fragrance Oil 5420492 | 6 |
| ALCOHOL 96.2% | 47.8 |
| UV BLEND | 2 |
| WATER | 4.5 |
| | (total water content ∼7.2 wt%) |

**Example 15:**

|  | wt% |
|---|---|
| ISODODECANE | 29 |
| IMP | 8.7 |
| REWOTERIC AM C | 2 |
| Fragrance Oil 5420492 | 6 |
| ALCOHOL 96.2% | 47.8 |
| UV BLEND | 2 |
| WATER | 4.5 |
| | (total water content ∼7.2 wt%) |

**Example 16:**

|  | wt% |
|---|---|
| ISODODECANE | 29 |
| IMP | 8.7 |
| REWOPOL SBFA 30 B | 2 |
| Fragrance Oil 5420492 | 6 |
| ALCOHOL 96.2% | 47.8 |
| UV BLEND | 2 |
| WATER | 4.5 |
| | (total water content ∼7.2 wt%) |

**Example 17:**

|  | wt% |
|---|---|
| ISODODECANE | 29 |
| IMP | 8.7 |
| PLANTACARE 818 UP | 2 |
| Fragrance Oil 5420492 | 6 |
| ALCOHOL 96.2% | 47.8 |
| UV BLEND | 2 |
| WATER | 4.5 |
| (total water content ~7.2 wt%) | |

**Example 18:**

|  | wt% |
|---|---|
| ISODODECANE | 29 |
| IMP | 6.31 |
| DPG | 4 |
| Fragrance Oil 173250 T | 6 |
| ALCOHOL 96.2% | 47.8 |
| UV BLEND | 2 |
| WATER | 4.5 |
| COLORANT | 0.39 |
| (total water content ~7.2 wt%) | |

**Example 19:**

|  | wt% |
|---|---|
| ISODODECANE | 29 |
| IMP | 6.31 |
| HYDROLITE 5 | 4 |
| Fragrance Oil 173250 T | 6 |
| ALCOHOL 96.2% | 47.8 |
| UV BLEND | 2 |
| WATER | 4.5 |
| COLORANT | 0.39 |
| (total water content ~7.2 wt%) | |

**Example 20:**

|  | wt% |
|---|---|
| ISODODECANE | 29 |
| IMP | 6.31 |
| ISOPENTYLDIOL | 4 |
| Fragrance Oil 173250 T | 6 |
| ALCOHOL 96.2% | 47.8 |
| UV BLEND | 2 |
| WATER | 4.5 |
| COLORANT | 0.39 |

16

(continued)

wt%
(total water content ~7.2 wt%)

**Example 21:**

|  | wt% |
|---|---|
| ISODODECANE | 29 |
| IMP | 6.31 |
| ISOPENTYLDIOL | 4 |
| Fragrance Oil 173250 T | 6 |
| ALCOHOL 96.2% | 47.8 |
| UV BLEND | 2 |
| WATER | 4.5 |
| COLORANT | 0.39 |
|  | (total water content ~7.2 wt%) |

**Example 22:**

|  | wt% |
|---|---|
| ISODODECANE | 29 |
| IMP | 6.31 |
| HYDAGEN CAT | 4 |
| Fragrance Oil 173250 T | 6 |
| ALCOHOL 96.2% | 47.8 |
| UV BLEND | 2 |
| WATER | 4.5 |
| COLORANT | 0,39 |
|  | (total water content ~7.2 wt%) |

**Example 23:**

|  | wt% |
|---|---|
| ISODODECANE | 29 |
| IMP | 6.31 |
| HYDROLITE 6 | 4 |
| Fragrance Oil 173250 T | 6 |
| ALCOHOL 96.2% | 47.8 |
| UV BLEND | 2 |
| WATER | 4.5 |
| COLORANT | 0.39 |
|  | (total water content ~7.2 wt%) |

**Example 24:**

|  | wt% |
|---|---|
| ISODODECANE | 29 |
| IMP | 6.31 |
| MMB | 4 |
| Fragrance Oil 173250 T | 6 |
| ALCOOL 96.2% | 47.8 |
| UV BLEND | 2 |
| WATER | 4.5 |
| COLORANT | 0.39 |

(total water content ~7.2 wt%)

**Example 25:**

|  | wt% |
|---|---|
| ISODODECANE | 29 |
| IMP | 6.31 |
| ISOPROPYL ADIPATE | 4 |
| Fragrance Oil 173250 T | 6 |
| ALCOHOL 96.2% | 47.8 |
| UV BLEND | 2 |
| WATER | 4.5 |
| COLORANT | 0.39 |

(total water content ~7.2 wt%)

**Example 26:**

|  | wt% |
|---|---|
| ISODODECANE | 29 |
| IMP | 6.31 |
| ISOBUTYL ADIPATE | 4 |
| Fragrance Oil 173250 T | 6 |
| ALCOHOL 96.2% | 47.8 |
| UV BLEND | 2 |
| WATER | 4.5 |
| COLORANT | 0.39 |

(total water content ~7.2 wt%)

**Example 27:**

|  | wt% |
|---|---|
| ISODODECANE | 29 |
| ISOHEXADECANE | 3 |
| DC YELLOW11 sol 0.1% in IHD | 1 |
| Fragrance Oil Skinscent B14 | 12 |
| Alcohol SDA 39C 190p | 49 |
| UV BLEND | 1 |

(continued)

| | wt% |
|---|---|
| WATER | 4.7 |
| FDC Red 4 sol. 0.2% in water | 0.3 |

(total water content ∼8.5 wt%)

**Example 28:**

| | wt% |
|---|---|
| LILAC | 29 |
| EO CK 072777NYBH | 7 |
| ALCOHOL 96.2% | 63.23 |
| UV BLEND 1A | 0.5 |
| SOL AC FD & C BLUE NO1 (0.29%) | 0.17 |
| SOL DC VIOLET 2 EXT (0.2%) | 0.1 |

(total water content ∼3.6%)

In this composition the oily phase formed a spherical/ oval shape within the water-alcohol phase.

**Example 29:**

| | wt% |
|---|---|
| Fragrance Oil 173250T | 6 |
| Isododecane | 30 |
| Pure Alcohol | 61 |
| Water | 3 |

In this composition no biphase was formed.

**Example 30:**

| | wt% |
|---|---|
| Fragrance Oil 173250T | 5.66 |
| Isododecane | 28.3 |
| Pure Alcohol | 57.55 |
| Water | 2.83 |
| Glycerin | 5.66 |

**Example 31:**

| | wt% |
|---|---|
| Fragrance Oil 173250T | 5.6 |
| Isododecane | 28.05 |
| Pure Alcohol | 57 |
| Water | 2.81 |

(continued)

|  | wt% |
|---|---|
| Propylene glycol | 6.54 |

**Example 32:**

|  | wt% |
|---|---|
| Fragrance Oil 173250T | 5.5 |
| Isododecane | 27.53 |
| Pure Alcohol | 55.96 |
| Water | 2.75 |
| PEG-4 | 8.26 |

**Example 33:**

|  | wt% |
|---|---|
| Fragrance Oil 173250T | 5.36 |
| Isododecane | 26.79 |
| Pure Alcohol | 54.46 |
| Water | 2.68 |
| DPG | 10.71 |

In this composition no biphase was formed.

**Example 34:**

|  | wt% |
|---|---|
| Isododecane | 50.00 |
| Pure alcohol | 50.00 |

In this composition no biphase was formed.

**Example 35:**

|  | wt% |
|---|---|
| Isododecane | 42.37 |
| Pure alcohol | 42.38 |
| DPG | 15.25 |

In this composition no biphase was formed.

**Example 36:**

|  | wt% |
|---|---|
| Isododecane | 43.63 |
| Pure alcohol | 43.63 |
| Propylene Glycol | 12.74 |

**Example 37:**

|  | wt% |
|---|---|
| Isododecane | 45.83 |
| Pure alcohol | 45.83 |
| Glycerin | 8.34 |

**Example 38:**

|  | wt% |
|---|---|
| Isododecane | 44.80 |
| Pure alcohol | 44.80 |
| PEG-4 | 10.40 |

**Example 39:**

|  | wt% |
|---|---|
| Isododecane | 44.60 |
| Pure alcohol | 44.60 |
| PEG-8 | 10.80 |

**Example 40:**

|  | wt% |
|---|---|
| Isododecane | 42.12 |
| Pure alcohol | 42.13 |
| Sorbitol 70 | 15.75 |

**Example 41:** Single fragrance migration study in different biphasic compositions

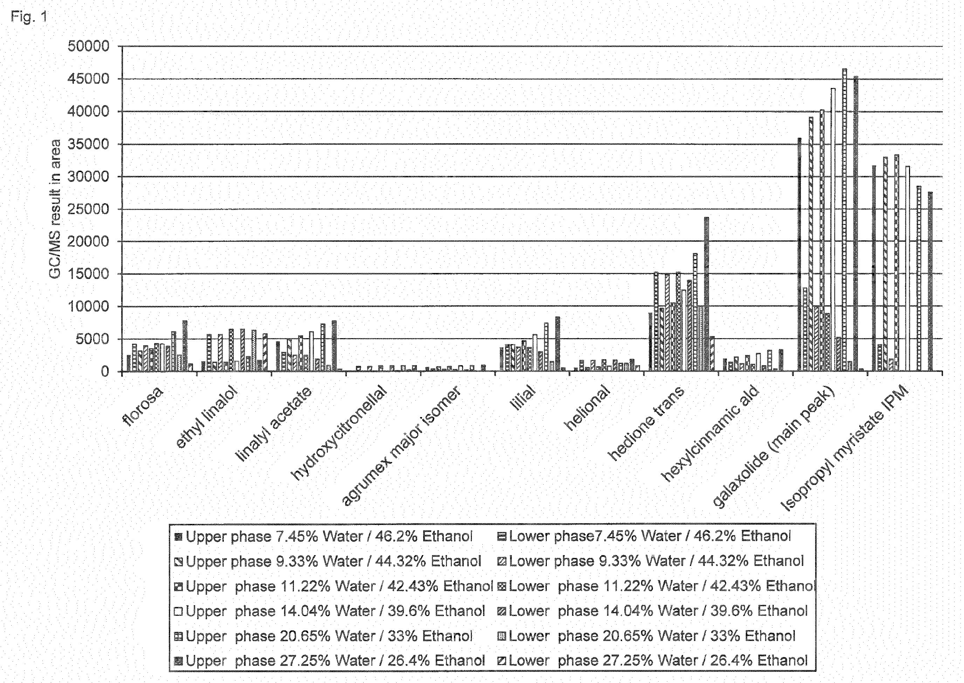

Fig. 1 illustrates that the water amount is critical to determine the partitioning of the fragrances. The different amounts of water ranging from ~7.4 wt% (together with ~46.2 wt% ethanol within the water-alcohol phase) to ~27.25 wt% (together with ~26.4 wt% ethanol within the water-alcohol phase) have an effect on the migration potential of the single components. Fig. 1 has been obtained using GC/MS analytical tool. Depending primary on the log P of fragrances and/or secondary of the chemical families/functions of those fragrances, modification of the polarity will have the effect of pushing the fragrance in one preferred phase of the biphasic composition. For example ethyl Linalool with a log P-value of 3.3 stays in the hydro-alcoholic phase when increasing the ratio of water. Contrary to that galaxolide with a log P-value of 6.2 and hexylcinnamic aldehyde with a log P-value of 4.8 which are migrating in the oily phase when the amount of water is increased. Fig. 1 also shows that non-aromatic solvents like Isopropyl Myristate (IPM) with a log P-value of 7.2 migrate following the same rules in the oil phase when modifying the polarity of the hydro-alcoholic phase.

To determine the distribution of first fragrance and second fragrance in the biphasic composition three formulations (regular EDT, biphasic composition with low amount of water in the water-alcohol phase, biphasic composition with high amount of water in the water-alcohol phase) were tested and compared using GC/MS analysis and Expert evaluator.

Regular EDT:

|  | wt% |
|---|---|
| Fragrance oil Giddy 11598/00 | 12% |
| Alcohol 96.2% | 76% |
| UV blend | 2% |
| Deionized Water | 10% |

(total water content ~4.33%)

**Biphasic composition with low amount of water in the bottom phase (i.e. water-alcohol phase):**

|  | wt% |
|---|---|
| Isododecane | 29% |
| Isopropyl myristate | 3.35% |
| Fragrance oil Giddy 11598/00 | 12% |
| Alcohol 96.2% | 49% |
| UV blend | 2% |
| Deionized Water | 4.65% |

(total water content ~7.4%)

**Biphasic composition with high amount of water in the bottom phase (i.e. water-alcohol phase):**

|  | wt% |
|---|---|
| Isododecane | 29% |
| Isopropyl myristate | 3.35% |
| Fragrance oil Giddy 11598/00 | 12% |
| Alcohol 96.2% | 28% |
| UV blend | 2% |
| Deionized Water | 25.65% |

(total water content ~27.24%)

**Fragrance oil Giddy 11598/00 distribution in the above formulations**

| Result in % based on GC/MS analysis | Regular EDT aromatics % | Bi phase formula low amount of water in the bottom phase | | Bi phase formula high amount of water in the bottom phase | |
|---|---|---|---|---|---|
| Aromatic Raw Material name | | Upper phase aromatics % | Lower phase aromatics % | Upper phase aromatics % | Lower phase aromatics % |
| florosa | 3.82 | 1.86 | 5.12 | 3.93 | 5.86 |
| ethyl linalol | 4.39 | 1.11 | 6.86 | 0.83 | 28.40 |
| linalyl acetate | 3.37 | 3.38 | 3.52 | 3.89 | 1.63 |
| hydroxycitronellal | 0.53 | 0.06 | 0.88 | 0.12 | 4.13 |
| agrumex major isomer | 0.41 | 0.45 | 0.40 | 0.47 | 0.16 |
| lilial | 3.92 | 2.70 | 4.90 | 4.18 | 2.43 |
| helional | 1.32 | 0.40 | 2.03 | 0.91 | 4.10 |
| hedione trans | 13.28 | 6.64 | 18.40 | 11.94 | 26.10 |
| hexylcinnamic ald | 1.54 | 1.43 | 1.65 | 1.67 | 0.46 |
| galaxolide (main peak) | 20.25 | 26.53 | 15.47 | 22.92 | 1.64 |
| Isopropyl Myristate IPM | 13.34 | 23.40 | 4.96 | 13.93 | 0.00 |
| Other aromatics not disclosed | 33.82 | 32.02 | 35.81 | 35.21 | 25.08 |
| | | | | | |
| total % | 100.00 | 100.00 | 100.00 | 100.00 | 100.00 |

| Expert evaluator olfactive results based on Fragrance oil Giddy 11598/00 | Regular EDT 12 % aromatics | Bi phase formula low amount of water in the bottom phase | | Bi phase formula high amount of water in the bottom phase | |
|---|---|---|---|---|---|
| | | Upper phase 14.3% aromatics | Lower phase 11.2% aromatics | Upper phase 22.2% aromatics | Lower phase 5.2% aromatics |
| Evaluation T 0-5min Top Note | Olfactive Reference | Slight Olfactive change compare to Reference but no character change | Slight Olfactive change compare to Reference but no character change | Slight Olfactive change compare to Reference with slight character change | Olfactive change compare to Reference providing a character change |
| Evaluation T 12h Bottom Note | Olfactive Reference | Slight Olfactive change compare to Reference but no character change | Slight Olfactive change compare to Reference but no character change | Slight Olfactive change compare to Reference with slight character change | Olfactive change compare to Reference providing a character change |

This example demonstrates that it is possible to formulate biphasic compositions to have first and second fragrance olfactively equal or to formulate biphasic compositions to have olfactively different first and second fragrances. This conclusion was made comparing the regular EDT and the biphasic composition used in this demonstration.

**Example 42:** Stability of individual fragrances of fragrance oil 173250 T within the different phases of the biphasic composition

ISODODECANE                           29

(continued)

| | |
|---|---|
| IMP | 10.31 |
| DC Yellow 11 sol 0.1% in IPM | 0.035 |
| DC RED 17 sol 0.1% in IPM | 0.145 |
| Fragrance Oil 173250 T | 6 |
| ALCOOL 96.2% | 47.8 |
| UV BLEND | 2 |
| WATER | 4.5 |
| FDC Yellow 5 sol. 0.2% in water | 0.21 |

(total water content ∼7.2 wt%)

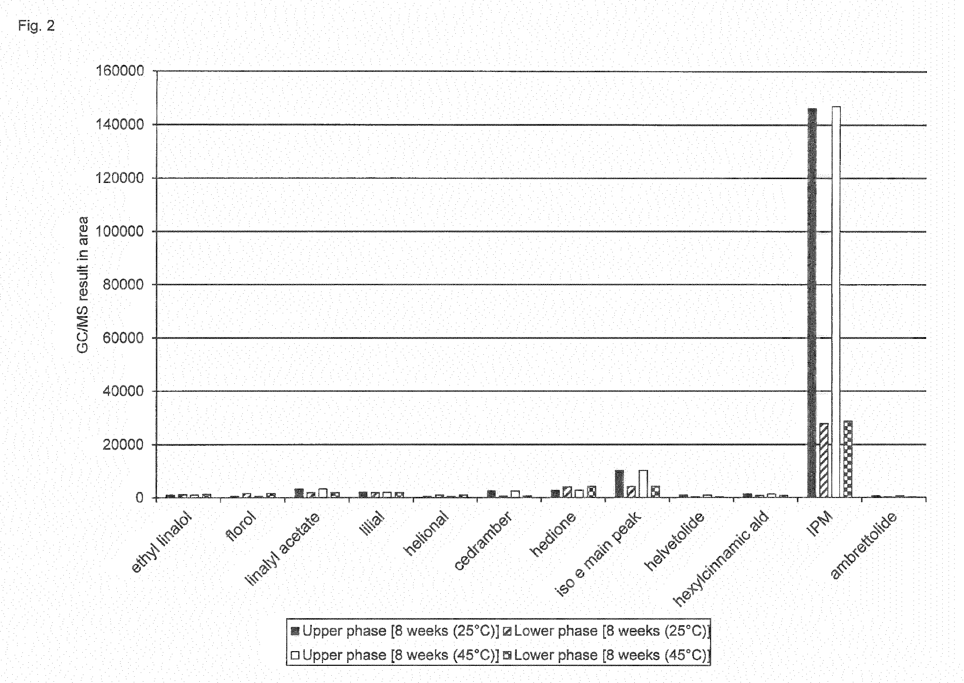

Fig. 2 demonstrates the good stability of the composition in aging tests. The distribution of the individual fragrances within the phases of the biphasic composition is stable after 8 weeks at different temperatures (25°C or 45°C).

**Claims**

1. Biphasic composition comprising or consisting of alcohol, a polar compound, a fragrance and a branched hydrocarbon.

2. Biphasic composition according to claim 1, wherein the biphasic composition comprises or consists of:

   20 to 75 wt% alcohol;
   0.5 to 30 wt% polar compound;
   1 to 50 wt% fragrance and
   5 to 60 wt% branched hydrocarbon,

   based on the total weight of the composition.

3. The biphasic composition according to claim 1 or 2, wherein the alcohol is contained in the composition in an amount of 20 to 75 wt%, preferably in an amount of 30 to 70 wt% and more preferred in an amount of 40 to 65 wt% based on the total weight of the composition.

4. The biphasic composition according to one of the preceding claims, wherein the alcohol is ethanol.

5. The biphasic composition according to one of the preceding claims, wherein the polar compound is contained in the composition in an amount of 0.5 to 30 wt%, preferably in an amount of 0.75 to 15 wt%, more preferred in an amount of 1 to 10 wt% and even more preferred in an amount of 2 to 9 wt% based on the total weight of the composition.

6. The biphasic composition according to one of the preceding claims, wherein the polar compound is water.

7. The biphasic composition according to one of the preceding claims, wherein the fragrance is contained in the composition in an amount of 1 to 50 wt%, preferably in an amount of 2 to 30 wt% and more preferred in an amount of 5 to 20 wt% based on the total weight of the composition.

8. The biphasic composition according to one of the preceding claims, wherein the branched hydrocarbon is contained in the composition in an amount of 5 to 60 wt%, preferably in an amount of 10 to 40 wt% and more preferred in an amount of 15 to 35 wt% based on the total weight of the composition.

9. The biphasic composition according to one of the preceding claims, wherein the branched hydrocarbon(s) comprises 5 to 20 carbon atoms, preferably 8 to 19 and more preferred 9 to 16 carbon atoms.

10. The biphasic composition according to one of the preceding claims, wherein the branched hydrocarbon is an alkane.

11. The biphasic composition according to claim 10, wherein the alkane is isododecane.

**12.** The biphasic composition according to one of the preceding claims, wherein the fragrance comprises at least a first fragrance and a second fragrance.

**13.** The biphasic composition according to claim 12, wherein the first fragrance and the second fragrance are predominantly in different phases of the composition.

**14.** The biphasic composition according to one of the preceding claims, wherein the composition comprises a colorant.

**15.** The biphasic composition according to claim 14, wherein the colorant comprises at least a first colorant and a second colorant.

**16.** The biphasic composition according to claim 15, wherein the first colorant and the second colorant are predominantly in different phases of the composition.

Fig. 1

Fig. 2

**EP 2 954 935 A1**

Europäisches
Patentamt
European
Patent Office
Office européen
des brevets

# EUROPEAN SEARCH REPORT

Application Number

EP 14 17 2212

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 127 632 A1 (COTY INC [US]) 2 December 2009 (2009-12-02) | 1,4-10, 12 | INV. A61Q13/00 A61K8/03 A61K8/31 A61K8/34 |
| Y | * page 6; example 3 * * page 7; example VI * ----- | 1-16 | |
| Y | DE 42 41 799 C1 (KAO CORP GMBH [DE]) 5 January 1994 (1994-01-05) * page 3 - page 4; examples 2,3 * ----- | 1-16 | |
| Y | US 2011/305656 A1 (GRANDJON VINCENT [FR]) 15 December 2011 (2011-12-15) * page 9; example 11 * ----- | 1-16 | |
| Y | EP 1 169 998 A2 (WELLA AG [DE]) 9 January 2002 (2002-01-09) * page 3, paragraph 10 * * page 14; claim 20 * ----- | 1-16 | |

| | | | TECHNICAL FIELDS SEARCHED (IPC) |
|---|---|---|---|
| | | | A61Q A61K |

The present search report has been drawn up for all claims

| Place of search | Date of completion of the search | Examiner |
|---|---|---|
| Munich | 17 November 2014 | Bader, Karl Günther |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

28

**ANNEX TO THE EUROPEAN SEARCH REPORT
ON EUROPEAN PATENT APPLICATION NO.** EP 14 17 2212

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2014

| Patent document cited in search report | | Publication date | Patent family member(s) | | Publication date |
|---|---|---|---|---|---|
| EP 2127632 | A1 | 02-12-2009 | AT | 532499 T | 15-11-2011 |
| | | | EP | 2127632 A1 | 02-12-2009 |
| | | | EP | 2181690 A1 | 05-05-2010 |
| | | | ES | 2376826 T3 | 20-03-2012 |
| DE 4241799 | C1 | 05-01-1994 | DE | 4241799 C1 | 05-01-1994 |
| | | | DE | 9216886 U1 | 14-04-1994 |
| | | | JP | 3243356 B2 | 07-01-2002 |
| | | | JP | H06279241 A | 04-10-1994 |
| US 2011305656 | A1 | 15-12-2011 | BR | PI1004511 A2 | 16-04-2013 |
| | | | CN | 102078263 A | 01-06-2011 |
| | | | EP | 2324816 A2 | 25-05-2011 |
| | | | FR | 2952531 A1 | 20-05-2011 |
| | | | US | 2011305656 A1 | 15-12-2011 |
| EP 1169998 | A2 | 09-01-2002 | AT | 346581 T | 15-12-2006 |
| | | | BR | 0102716 A | 26-02-2002 |
| | | | DE | 10033414 A1 | 24-01-2002 |
| | | | EP | 1169998 A2 | 09-01-2002 |
| | | | US | 2002031478 A1 | 14-03-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130209385 A1

- WO 2013012939 A1

**Non-patent literature cited in the description**

- International Cosmetic Ingredient Dictionary and Handbook. 2008, vol. 3, 3193-3200